# FASCICULE DE BREVET EUROPEEN

(11) **EP 1 566 147 B1**
(45) Date de publication et mention de la délivrance du brevet: **19.12.2018**
(21) Numéro de dépôt: 05003514.6
(22) Date de dépôt: 18.02.2005
(51) Int. Cl.: A61B 17/16

(54) **Ancillaire destiné à la préparation d'un fût fémoral en vue d'une arthroplastie de hanche mini-invasive**
Hilfsinstrument zur Vorbereitung des Femurs für eine minimal-invasive Hüftarthroplastik
Ancillary for the preparation of the femur for a minimally invasive hip arthroplasty

(30) Priorité: 23.02.2004 FR 0401973
(43) Date de publication de la demande: 24.08.2005
(73) Titulaire: Zimmer France, 25461 Etupes Cedex (FR); Henky, Pierre, 67115 Plobsheim (FR)
(72) Inventeur: Henky, Pierre, 25A, rue Proudhon 25000 Besançon (FR); Bouraly, Jean Pierre, 25A, rue Proudhon 25000 Besançon (FR)
(74) Mandataire: Mays, Julie

(56) Documents cités:
- EP-A- 0 359 097
- EP-A- 0 766 948
- EP-A- 1 226 786

## Description

La présente invention concerne un ancillaire destiné à la préparation d'un fût fémoral en vue de la pose d'une tige fémorale cimentée ou sans ciment à « voie d'abord » dite mini-invasive.

Les « voies d'abord » antérieures, postérieures ont été décrites par de nombreux chirurgiens telles les voies de Hardinge, Moore.

Ces « voies d'abord » sont suffisament larges pour exposer le fémur et utiliser des râpes d'un seul tenant, avec un manche monobloc ou bi-bloc.

Ces râpes ont pour but de pouvoir réaliser un logement ajusté dans le fût fémoral pour pouvoir bloquer à force la tige.

Cette tige pourra être fixée à l'aide d'un ciment par blocage.

Au lieu de procéder à une incision relativement large (15 à 25cm), la technique mini-invasive permet de se contenter d'une petite incision.

Les avantages de cette nouvelle technique, quelle que soit la prothèse, sont de minimiser les dommages infligés lors d'une intervention chirurgicale, quelle qu'elle soit, aux muscles, tendons et ligaments.

Ainsi, au lieu de couper les fibres musculaires, cette nouvelle technique permet de passer entre les muscles eux-mêmes ou au pire au travers des fibres musculaires en les séparant.

Après l'intervention, les muscles touchés reprennent leur forme et leur fonction initiale beaucoup plus rapidement que s'ils avaient été coupés.

Les effets bénéfiques sont :
- des pertes sanguines moindres,
- des douleurs moindres,
- une récupération beaucoup plus rapide,
- une stabilité de l'articulation plus grande, diminuant le risque de luxation,
- une rééducation facilitée et des risques d'infection réduits.

Parmi les différentes « voies d'abord » proposant cette technique minimale invasive, la voie antéro-externe dérivée de WATSON-JONES, modifiée dans son orientation, permet une exposition du fémur après avoir réalisé la coupe col fémorale remarquable.

Une technique de ce type permet l'utilisation de tiges cimentées ou non cimentées, toutes les autres techniques permettant de l'usage de tiges sans ciment.

Afin de pouvoir exposer convenablement l'articulation, des écarteurs, des fraises cotyloïdiennes ayant des géométries particulières, on permis de travailler l'acétabulum et le fémur.

La technique opératoire originale proposée présente la possibilité d'écarter le moyen fessier grâce à une curarisation, ce qui permet d'avoir un « abord » fémoral dans lequel il sera possible d'introduire les râpes.

Après la mise en place d'un petit alésoir, il est nécessaire de préparer le logement du futur implant.

Il a déjà été décrit des râpes d'un seul tenant qui permettent actuellement de préparer le fût fémoral.

Lors de l'introduction d'une râpe traditionnelle dans le fût fémoral, il est impossible dans une « voie d'abord » antéro-externe mini-invasive d'éviter de léser fortement le moyen fessier et les autres tissus mous adjacents sans risquer en plus de faire une fausse route et de préparer le fût fémoral en prédisposant la pose à un varus.

Il est connu par le brevet européen EP 0 766 948 de disposer de deux rapes dont l'une a une fonction d'ébauchoir destinée à la partie proximale du fût fémoral se terminant par un embout lui donnant une longueur sensiblement égale à une seconde rape destinée à préparer la partie distale du fût fémoral.

Cette seconde rape présente une partie active dans sa partie distale présentant l'inconvénient de léser les muscles des voies d'abord antérieures, provoquant des suites opératoires compliquées, des pertes de sang et des risques de luxation.

La présente invention a pour but de remédier à ces inconvénients et concerne à cet effet un ancillaire destiné à la préparation d'un fût fémoral en vue de la pose d'une tige fémorale cimentée ou sans ciment à « voie d'abord » dite mini-invasive, caractérisé en ce qu'il est constitué par deux râpes :
- dont la première a une fonction d'ébauchoir destinée à préparer la partie proximale du fût fémoral et qui présente une partie active correspondant sensiblement au tiers supérieur de la tige fémorale à implanter, et
- dont la seconde intervenant successivement à la première a une fonction destinée à préparer la partie distale du même fût fémoral et présente une partie active correspondant sensiblement aux deux tiers inférieur de la tige fémorale à implanter, alors que le tiers supérieur optimal de cette même seconde râpe présente une partie lisse et affinée et correspond sensiblement à la partie active de la première râpe ayant déjà intervenue dans la partie proximale du fût fémoral, de manière à permettre à la dite seconde râpe d'opérer dans la partie distale du fût fémorale en évitant de léser les muscles.

Cette description donnée à titre d'exemple non limitatif, fera mieux comprendre comment l'invention peut être réalisée en référence aux dessins annexés sur lesquels :
La figure 1 représente une vue en plan d'une première râpe « partie proximale ».
La figure 1A est une vue en coupe transversale selon la ligne IA, IA selon la figure 1.
La figure 2 est une vue de côté de la râpe selon la figure 1.
La figure 3 est une vue en plan d'une première râpe « partie distale ».
La figure 3A est une vue en coupe transversale selon ligne IIIA, IIIA selon la figure 3.
La figure 4 est une vue de côté de la râpe selon la figure 3.
La figure 5 est une vue à échelle agrandie montrant le profil des dents tant de la râpe selon les figures 1 et 2 que de la râpe selon les figures 3 et 4.

L'ancillaire représenté sur l'ensemble des figures est destiné à la préparation d'un fût fémoral, en vue de la pose d'une tige fémorale cimentée ou sans ciment à « voie d'abord » dite mini-invasive, comme déjà décrite dans le préambule.

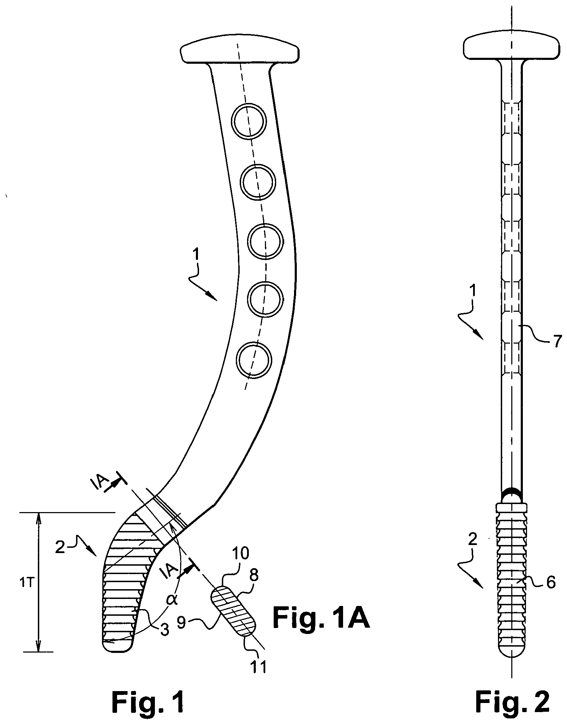

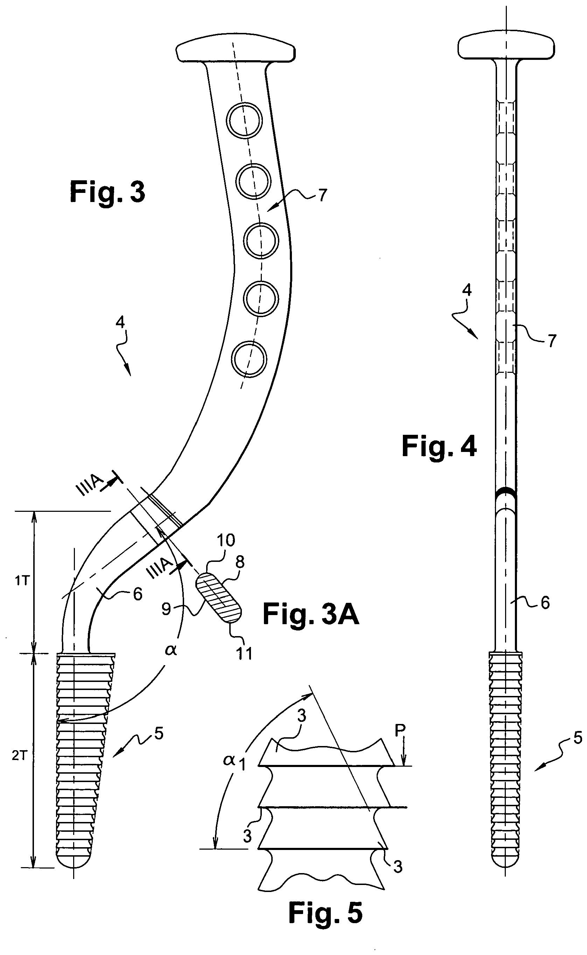

Selon l'invention, cet ancillaire est constitué par deux râpes dont l'une est représentées sur les figures 1, 1A et 2 et l'autre sur les figures 3, 3A et 4.

La première râpe 1 a une fonction d'ébauchoir et est destinée à préparer la partie proximale du fût fémoral.

Elle présente une partie active 2 comportant des dents 3, correspondant sensiblement au tiers supérieur 1T de la tige fémorale à implanter.

Cet ébauchoir permet de dégager en particulier le grand trochanter.

Par le fait de sa faible longueur, il est beaucoup plus facile, par des mouvements de torsion d'engager cette râpe 2 sans léser les muscles, puisque c'est seulement dans les deux premiers centimètres que l'on pourra avoir un engagement fixe et l'effet de râpe.

Cette râpe proximale ou ébauchoir 2, par sa faible longueur, permet de travailler la partie proximale du fémur avec des angulations possibles allant de l'axe mécanique fémoral à l'axe du col.

La seconde râpe 4, intervenant successivement à la première 1, a une fonction destinée à préparer la partie distale du même fût fémoral et présente une partie active 5 correspondant sensiblement aux deux tiers inférieurs 2T de la tige fémorale à implanter, alors que le tiers supérieur proximal 1T de cette même seconde râpe 5 présente une partie lisse et affinée et correspond sensiblement à la partie active 2 de la première râpe 1, ayant déjà intervenue dans la partie proximale du fût fémoral.

De cette manière, on permet à la seconde râpe 5 d'opérer dans la partie distale du fût fémoral en évitant de léser les muscles.

Le faible encombrement obtenu permet d'éviter les fausses routes.

L'ensemble de l'ancillaire constitue un système efficace pour éviter une pause en varus pour avoir une bonne précision du logement fémoral, pour éviter de léser les muscles, de les cisailler, en particulier dans cette « voie d'abord ».

Le relief des râpes est indifférent. Seule la forme de la partie lisse 6 qui surplombe les parties coupantes 5 de la râpe distale 4 doit être lisse.

La forme de la partie distale 5 de la râpe 4 est optimisée, ainsi que le matériau choisi, pour avoir une bonne résistance mécanique suffisante et éviter tout risque de rupture.

Il est à noter que l'une ou l'autre des râpes 1 ou 4 comportent un manche 7 identique, de forme adaptée à une voie antérieure ou à une voie postérieure.

Selon le présent exemple de réalisation représenté sur les figures, il s'agit de râpes adaptées à une voie antérieure.

Les râpes 1, 4 peuvent aussi bien être des râpes prothèses d'essai ou formant avec leur manche respectif 7 des éléments monoblocs.

Préférentiellement, les râpes 1, 4 sont réalisées en acier inoxydable.

L'utilisation de l'acier inoxydable ou d'autre type de matériaux purs à pour but d'éviter l'usure des dents 3 desdites râpes.

Comme le montre aussi bien les figures 1A que 3A, la section transversale tant des râpes 1, 4 que de leur manche 7 s'inscrit dans un rectangle et est formée de deux plans parallèles 8, 9 reliés entre eux par deux côtés longitudinaux arrondis 10, 11.

On notera toutefois que la longueur de la section de la râpe 4 distale (voir figure 3A) est plus importante que celle de la section (voir figure 1A) de la partie proximale de l'ébauchoir 2.

Selon une autre caractéristique de l'invention, l'une ou l'autre des râpes 1, 4 le constituant forme avec la base de leur manche respectif 7 un angle α d'environ 130°.

Comme le montre bien les figures, le manche 7 prolongeant soit directement la râpe 2 ou indirectement la râpe 5, prolongée de la partie lisse 6, est incurvé selon un rayon orienté selon une direction opposée, par rapport à la râpe 2 ou 5.

Comme le montre la figure 5, l'angle d'attaque des dents 3 des râpes 1 ou 4, forme un angle α 1 de 65°.

Leur pas « p » est de 3,5 mm alors que leur rayon « r » de liaison d'une dent à l'autre est de 0,8 mm.

## Revendications

1. Ancillaire destiné à la préparation d'un fût fémoral en vue de la pose d'une tige fémorale cimentée ou sans ciment à « voie d'abord » dite mini-invasive, ledit ancillaire étant constitué par deux râpes :
- dont la première (1) a une fonction d'ébauchoir destinée à préparer la partie proximale du fût fémoral et qui présente une partie active (2) correspondant sensiblement au tiers supérieur (1T) de la tige fémorale à implanter, et
- dont la seconde (4) intervenant successivement à la première (1) a une fonction destinée à préparer la partie distale du même fût fémoral et présente une partie active (5) correspondant sensiblement aux deux tiers inférieur (2T) de la tige fémorale à implanter, alors que le tiers supérieur (1T) de cette même seconde râpe (5) présente une partie lisse et affinée (6) par rapport à la partie active (5), et correspond sensiblement à la partie active (2) de la première râpe (1) ayant déjà intervenue dans la partie proximale du fût fémoral, de manière à permettre à la dite seconde râpe (5) d'opérer dans la partie distale du fût fémorale en évitant de léser les muscles.

2. Ancillaire selon la revendication 1, **caractérisé en ce que** l'une ou l'autre des râpes (1, 4) le constituant comporte un manche (7) de forme adaptée à une voie antérieure ou à une voie postérieure.

3. Ancillaire selon les revendications 1 ou 2, **caractérisé en ce que** les râpes (1, 4) le constituant sont des râpes prothèses d'essai.

4. Ancillaire selon les revendications 1 ou 2, **caractérisé en ce que** les râpes (1, 4) le constituant forment avec leur manche respectif (7) des éléments monoblocs.

5. Ancillaire selon les revendications 1 à 4, **caractérisé en ce que** les râpes (1, 4) le constituant sont réalisées en acier inoxydable.

6. Ancillaire selon l'une des revendications 1 à 5, **caractérisé en ce que** la section transversale tant des râpes (1, 4) que de leur manche (7) s'inscrit dans un rectangle et est formée de deux plans parallèles (8, 9) reliés entre eux par deux côtés longitudinaux arrondis (10, 11).

7. Ancillaire selon l'une des revendications 1 à 6, **caractérisé en ce que** l'une ou l'autre des râpes (1, 4) le constituant forme avec la base de leur manche respectif (7) un angle α d'environ 130°.

## Patentansprüche

1. Hilfsinstrument zur Vorbereitung eines Oberschenkelschafts für die Einsetzung eines zementierten oder zementfreien Oberschenkelstiels mit einem "minimal-invasiv" genannten Zugang, wobei das Hilfsinstrument aus zwei Raspeln besteht:
- wobei die erste (1) eine Schruppfunktion aufweist, die dazu bestimmt ist, den proximalen Teil des Oberschenkelschafts vorzubereiten, und die einen aktiven Teil (2) aufweist, der im Wesentlichen dem oberen Drittel (1T) des zu implantierenden Oberschenkelstiels entspricht, und
- wobei die zweite (4), die nach der ersten (1) interveniert, eine Funktion aufweist, die dazu bestimmt ist, den distalen Teil desselben Oberschenkelschafts vorzubereiten, und einen aktiven Teil (5) aufweist, der im Wesentlichen den unteren zwei Dritteln (2T) des zu implantierenden Oberschenkelstiels entspricht,
während das obere Drittel (1T) dieser zweiten Raspel (5) einen glatten und verfeinerten Teil (6) in Bezug auf den aktiven Teil (5) aufweist, und im Wesentlichen dem aktiven Teil (2) der ersten Raspel (1) entspricht, die bereits in den proximalen Teil des Oberschenkelschafts eingegriffen ist, sodass die zweite Raspel (5) im distalen Teil des Oberschenkelschafts betrieben werden kann, ohne die Muskeln zu beschädigen.

2. Hilfsinstrument nach Anspruch 1, **dadurch gekennzeichnet, dass** die eine oder andere der Raspeln (1, 4), die die Komponente bilden, einen Griff (7) mit einer an einen vorderen oder hinteren Weg angepassten Form umfasst.

3. Hilfsinstrument nach den Ansprüchen 1 oder 2, **dadurch gekennzeichnet, dass** die Raspeln (1, 4), aus denen es besteht, Versuchsprothesenraspeln sind.

4. Hilfsinstrument nach Anspruch 1 oder 2, **dadurch gekennzeichnet, dass** die Raspeln (1, 4), aus denen es besteht, mit ihrem jeweiligen Griff (7) Monoblockelemente bilden.

5. Hilfsinstrument nach den Ansprüchen 1 bis 4, **dadurch gekennzeichnet, dass** die Raspeln (1, 4), aus denen es besteht, aus Edelstahl gefertigt sind.

6. Hilfsinstrument nach einem der Ansprüche 1 bis 5, **dadurch gekennzeichnet, dass** der Querschnitt sowohl der Raspeln (1, 4) als auch ihres Griffs (7) in einem Rechteck liegt und durch zwei parallele Ebenen (8, 9) gebildet ist, die durch zwei abgerundete Längsseiten (10, 11) miteinander verbunden sind.

7. Hilfsinstrument nach einem der Ansprüche 1 bis 6, **dadurch gekennzeichnet, dass** die Raspeln (1, 4), aus denen es besteht, mit der Basis ihres jeweiligen Griffs (7) einen Winkel α von etwa 130° bilden.

## Claims

1. Instrument intended for the preparation of a femoral shaft in order to fit a femoral stem, with or without cement, by a mini-invasive approach, said instrument consisting of two rasps:
- the first (1) of which has a shaping function intended to prepare the proximal portion of the femoral shaft and which has an active portion (2) corresponding substantially to the upper third (1T) of the femoral stem to be implanted, and
- the second (4) of which, following the first (1), performs a function intended to prepare the distal portion of the same femoral shaft and has an active portion (5) corresponding substantially to the lower two thirds (2T) of the femoral stem to be implanted, whereas the upper third (1T) of this same second rasp (5) has a smooth portion and is narrower (6) compared with the active portion (5), and corresponds substantially to the active portion (2) of the first rasp (1), said first rasp having already operated in the proximal portion of the femoral shaft in such a way that it allows the said second rasp (5) to operate in the distal portion of the femoral shaft, avoiding damage to the muscles.

2. Instrument according to claim 1, **characterised in that** either rasp (1, 4) constituting it has a shaft (7) shaped so that it is suitable for an anterior approach or a posterior approach.

3. Instrument according to claims 1 or 2, **characterised in that** the rasps (1, 4) constituting it are rasps for trial prostheses.

4. Instrument according to claims 1 or 2, **characterised in that** the rasps (1, 4) constituting it form one-piece elements with their respective shaft (7).

5. Instrument according to claims 1 to 4, **characterised in that** the rasps (1, 4) constituting it are made of stainless steel.

6. Instrument according to any one of claims 1 to 5, **characterised in that** the cross section both of the rasps (1, 4) as well as their shaft (7) fits into a rectangle and is formed by two parallel planes (8, 9) connected by two rounded longitudinal sides (10, 11) between them.

7. Instrument according to any one of claims 1 to 6, **characterised in that** either rasp (1, 4) constituting it forms an angle α of approximately 130° with the base of their respective shaft (7).
